# FASCICULE DE BREVET EUROPEEN

(11) **EP 2 338 466 B1**
(45) Date de publication et mention de la délivrance du brevet: **08.03.2017**
(21) Numéro de dépôt: 10195542.5
(22) Date de dépôt: 17.12.2010
(51) Int. Cl.: A61K 8/31, A61K 8/898, A61K 8/92, A61Q 5/00, A61Q 5/12

(54) **Composition cosmétique comprenant au moins un alcane linéaire volatil, au moins une silicone aminée de teneur particulière et au moins une huile végétale**
Kosmetische Zusammensetzung, die mindestens ein flüchtiges lineares Alkan, mindestens ein Aminsilikon mit speziellem Gehalt und mindestens ein Pflanzenöl enthält
Cosmetic composition containing at least one volatile linear alkane, at least one amine silicone having a particular content, and at least one vegetable oil

(30) Priorité: 23.12.2009 FR 0959526
(43) Date de publication de la demande: 29.06.2011
(73) Titulaire: L'Oréal, 75008 Paris (FR)
(72) Inventeur: Desenne, Patricia, 74370, Pringy (FR); Chesneau, Laurent, 92300 Levallois (FR)
(74) Mandataire: Dossmann, Gérard

(56) Documents cités:
- EP-A1- 2 074 986
- EP-A1- 2 111 847
- EP-A2- 1 169 998
- WO-A1-2007/052845
- FR-A1- 2 804 014
- FR-A1- 2 831 800
- FR-A1- 2 920 969
- FR-A1- 2 926 990
- US-A- 3 818 105
- US-B1- 6 723 309

## Description

La présente invention concerne une composition cosmétique comprenant un ou plusieurs alcane(s) linéaire(s) volatil(s), une ou plusieurs silicone(s) aminée(s) en une de teneur particulière, et une ou plusieurs une huile(s) végétale(s), son utilisation pour le traitement cosmétique des matières kératiniques, de préférence des fibres kératiniques comme les cheveux et un procédé de traitement cosmétique des matières kératiniques mettant en oeuvre ladite composition.

Dans le domaine du traitement capillaire, l'utilisation de solvants volatils est connue dans des produits capillaires de soins rincés ou non rincés. Ils sont généralement utilisés pour différentes raisons. Ils permettent notamment de modifier le rendu sensoriel d'un produit capillaire en lui conférant une texture légère et non collante dans la main. Ils peuvent également lui conférer un caractère glissant qui facilite la répartition du produit sur les cheveux et en particulier sur des cheveux secs.

Dans les émulsions aqueuses de type huile-dans-eau, qui peuvent se présenter sous forme de crèmes plus ou moins gélifiées, l'ajout de solvants volatils peut également permettre de solubiliser des gommes de silicone qui, de par leur viscosité intrinsèque, seraient difficiles à introduire dans les compositions.

Ces solvants volatils qui sont généralement des esters gras liquides, des huiles hydrocarbonées de type isododécane ou isohexadécane, et/ou des huiles siliconées peuvent notamment induire des problèmes de toucher gras, de manque de brillance, de cheveux raidis, durs.

Il subsiste donc un besoin de remplacer ces solvants volatils pour éviter les inconvénients cités ci-dessus.

La demanderesse a maintenant découvert de façon inattendue et surprenante, que l'association d'un ou plusieurs alcanes linéaires volatils, d'une ou plusieurs silicones aminées en une teneur particulière, et d'une ou plusieurs huiles végétales différentes des alcanes linéaires volatils permettait d'éviter les inconvénients mentionnés ci-dessus et d'améliorer les propriétés cosmétiques telles que le lissage, la souplesse, le démêlage, le volume en particulier par décollement des racines, et la tonicité de la chevelure.

En particulier, la composition selon l'invention permet d'obtenir des cheveux plus lisses, homogènes et/ou plus souples, au moment du rinçage. Sur cheveux humides, on obtient des cheveux plus faciles à démêler ou plus toniques et/ou ayant des racines plus décollées (en racines, les cheveux ne sont pas plaqués sur le cuir chevelu mais forment un angle, d'où apport de volume). Les cheveux traités par la composition selon l'invention sèchent rapidement. En outre, les cheveux secs sont plus souples et/ou plus lisses au toucher.

Ainsi, l'invention a pour objet une composition cosmétique comprenant, dans un milieu cosmétiquement acceptable :
- un ou plusieurs alcanes linéaires volatils, comportant de 7 à 15 atomes de carbone
- au moins 0,5% en poids d'une ou plusieurs silicones aminées, par rapport au poids total de la composition cosmétique,
- 3 à 20 % en poids d' une ou plusieurs huiles végétales différentes des alcanes linéaires volatils.

Elle a encore pour objet l'utilisation d'une composition selon l'invention pour le traitement cosmétique des matières kératiniques, de préférence des fibres kératiniques comme les cheveux, notamment comme produit capillaire de soin rincé.

Un autre objet de l'invention est un procédé de traitement cosmétique des matières kératiniques, de préférence des fibres kératiniques comme les cheveux, mettant en oeuvre ladite composition.

Par « un ou plusieurs alcane(s) linéaire(s) volatil(s) », on entend indifféremment « une ou plusieurs huile(s) alcane(s) linéaire(s) volatile(s) ».

Un alcane linéaire volatil convenant à l'invention est liquide à température ambiante (environ 25 °C) et sous pression atmosphérique (101 325 Pa ou 760 mm Hg).

Par « alcane linéaire volatil » convenant à l'invention, on entend un alcane linéaire, susceptible de s'évaporer au contact de la peau en moins d'une heure, à température ambiante (25°C) et sous pression atmosphérique (101 325 Pa), liquide à température ambiante, ayant notamment une vitesse d'évaporation allant de 0,01 à 15 mg/cm²/min, à température ambiante (25°C) et sous pression atmosphérique (101 325 Pa).

De préférence, le ou les alcanes linéaires volatils convenant à l'invention présentent une vitesse d'évaporation allant de 0,01 à 3,5 mg/cm²/min, mieux de 0,01 à 1,5 mg/cm²/min, à température ambiante (25°C) et sous pression atmosphérique (101 325 Pa).

De façon plus préférée, le ou les alcanes linéaires volatils convenant à l'invention présentent une vitesse d'évaporation allant de 0,01 à 0,8 mg/cm²/min, préférentiellement de 0,01 à 0,3 mg/cm²/min, et encore plus préférentiellement de 0,01 à 0,12 mg/cm²/min, à température ambiante (25°C) et sous pression atmosphérique (101 325 Pa).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m³ régulée en température (25 °C) et en hygrométrie (humidité relative 50 %), 15 g de solvant hydrocarboné volatil.

On laisse le solvant hydrocarboné volatil s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm²) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm²) et par unité de temps (minute).

Selon un mode de réalisation préféré, le ou les alcanes linéaires volatils convenant à l'invention ont une pression de vapeur (appelée également pression de vapeur saturante) non nulle, à température ambiante, en particulier une pression de vapeur allant de 0,3 Pa à 6000 Pa.

De façon préférée, le ou les alcanes linéaires volatils convenant à l'invention ont une pression de vapeur allant de 0,3 à 2000 Pa, mieux de 0,3 à 1000 Pa, à température ambiante (25°C).

De façon plus préférée, le ou les alcanes linéaires volatils convenant à l'invention ont une pression de vapeur allant de 0,4 à 600 Pa, préférentiellement de 1 à 200 Pa, et encore plus préférentiellement de 3 à 60 Pa, à température ambiante (25°C).

Selon un mode de réalisation, un alcane linéaire volatil convenant à l'invention peut présenter un point éclair compris dans l'intervalle variant de 30 à 120 °C, et plus particulièrement de 40 à 100 °C. Le point éclair est en particulier mesuré selon la Norme iso 3679.

Selon un mode de réalisation, le ou les alcanes linéaires volatils convenant dans l'invention peuvent être les alcanes linéaires comportant de 7 à 15 atomes de carbone, de préférence de 8 à 14 atomes de carbone, et mieux de 9 à 14 atomes de carbone.

De façon plus préférée, le ou les alcanes linéaires volatils convenant dans l'invention comprennent de 10 à 14 atomes de carbone, et encore plus préférentiellement de 11 à 14 atomes de carbone.

Le ou les alcanes linéaires volatils convenant à l'invention peuvent être avantageusement d'origine végétale.

De préférence, l'alcane linéaire volatil ou le mélange d'alcanes linéaires volatils présent dans la composition selon l'invention comprend au moins un isotope ¹⁴C du carbone (carbone 14). En particulier, l'isotope ¹⁴C peut être présent en un ratio isotopique ¹⁴C/¹²C (en nombre d'isotopes) supérieur ou égal à 1.10⁻¹⁶, de préférence supérieur ou égal à 1.10⁻¹⁵, de préférence encore supérieur ou égal 7,5.10⁻⁴, et mieux supérieur ou égal 1,5.10⁻¹³. De préférence, le ratio isotopique ¹⁴C / ¹²C va de 6.10⁻¹³ à 1,2.10⁻¹².

La quantité d'isotopes ¹⁴C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane ou mélange d'alcanes peut être obtenu, directement ou en plusieurs étapes, à partir d'une matière première végétale comme une huile, un beurre, une cire, etc.

A titre d'exemple d'alcanes convenant à l'invention, on peut mentionner les alcanes décrits dans les demandes de brevets WO 2007/068371 et WO2008/155059. Ces alcanes sont obtenus à partir d'alcools gras, eux-mêmes obtenus à partir d'huile de coprah ou de palme.

Le document FR 2831800 concerne des nouvelles compositions cosmétiques capillaires qui apportent des bonnes propriétés de lissage, légèreté, brillance, volume et démêlage des fibrres köratiniques.

A titre d'exemples d'alcanes linéaires convenant à l'invention, on peut citer le n-heptane (C7), le n-octane (C8), le n-nonane (C9), le n-décane (C10), le n-undécane (C11), le n-dodécane (C12), le n-tridécane (C13), le n-tétradecane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges, et mieux encore parmi le n-undécane, le n-tridécane, et leurs mélanges.

Selon un mode de réalisation préféré, on peut citer les mélanges de n-undécane (C11) et de n-tridécane (C13), en particulier obtenus notamment aux exemples 1 et 2 de la demande WO2008/155059.

On peut également citer le n-dodécane (C12) et le n-tétradécane (C 14) vendus respectivement sous les références PARAFOL 12-97 et PARAFOL 14-97 par la société Sasol, ainsi que leurs mélanges.

Un mode de réalisation consiste à utiliser un seul alcane linéaire volatil.

Alternativement, on peut utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C10/C11, C11/C12, ou C12/C13.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et le mélange C11/C13 pour un nombre de carbone n impair.

Selon un mode de réalisation préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C11/C13 ou un mélange d'alcanes linéaires volatils C12/C14. Avantageusement, on préfère utiliser un mélange de n-undécane et n-tridécane.

D'autres mélanges associant plus de 2 alcanes linéaires volatils selon l'invention, tels que, par exemple, un mélange d'au moins 3 alcanes linéaires volatils comportant de 7 à 15 atomes de carbone distincts et différant entre eux d'un nombre de carbone d'au moins 1, peuvent être utilisés dans l'invention.

Dans le cas des mélanges de deux alcanes linéaires volatils, lesdits deux alcanes linéaires volatils représentent de préférence plus de 95%, et mieux plus de 99% en poids du mélange.

Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil en Cₙ avec n allant de 7 à 15,
- de 10 à 50% en poids, de préférence de 20 à 45% en poids, de préférence de 24 à 40% en poids, d'alcane linéaire volatil en Cₙ₊ₓ avec x supérieur ou égal à 1, de préférence x=1 ou x=2, avec n+x compris entre 8 et 14,
par rapport au poids total des alcanes dans ledit mélange.

En particulier, ledit mélange d'alcanes linéaires volatils peut contenir en outre :
- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0,1% en poids d'hydrocarbures insaturés,
lesdits pourcentages étant exprimés par rapport au poids total du mélange.

Plus particulièrement, les alcanes linéaires volatils convenant dans l'invention peuvent être mis en oeuvre sous la forme d'un mélange n-undécane/n-tridécane.

En particulier, on utilisera un mélange d'alcanes linéaires volatils comprenant :
- de 55 à 80% en poids, de préférence de 60 à 75% en poids d'alcane linéaire volatil en C11 (n-undécane) et
- de 20 à 45% en poids, de préférence de 24 à 40% en poids d'alcane linéaire volatil en C13 (n-tridécane),
par rapport au poids total des alcanes dans ledit mélange.

Selon un mode de réalisation particulier, le mélange d'alcanes est un mélange n-undécane/n-tridécane, En particulier, un tel mélange peut être obtenu selon l'exemple 1 ou l'exemple 2 de la demande WO 2008/155059. L'utilisation d'un tel mélange confère des propriétés cosmétiques particulièrement avantageuses aux fibres kératiniques traitées, notamment en terme de souplesse et de volume.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane, de préférence dans un rapport 85/15 tel que le mélange commercialisé sous la dénomination VEGELIGHT 1214 par la société Biosynthis.

La composition de l'invention comprend de préférence de 0,5% à 90% en poids d'alcane(s) linéaire(s) volatil(s), en particulier de 1% à 50% en poids, plus particulièrement de 3% à 40%, et mieux encore de 3% à 30% en poids d'alcane(s) linéaire(s) volatil(s), par rapport au poids total de la composition.

Comme indiqué précédemment, la composition selon la présente invention contient au moins 0,5% en poids d'une ou plusieurs silicones aminées, par rapport au poids total de la composition.

Par « silicone aminée », on désigne tout polyaminosiloxane, c'est-à-dire tout polysiloxane comportant au moins une fonction amine primaire, secondaire, tertiaire ou un groupement ammonium quaternaire.

De préférence, la ou les silicones aminées utilisées dans la composition cosmétique selon la présente invention sont choisies parmi :
(a) les composés répondant à la formule (I) suivante :

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (I)

   dans laquelle :
   T est un atome d'hydrogène, ou un radical phényle, hydroxyle (-OH), ou alkyle en C₁-C₈, et de préférence méthyle ou alcoxy en C₁-C₈, de préférence méthoxy,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R¹ est un radical monovalent de formule -C_{q}H_{2q}L dans laquelle q est un nombre de 2 à 8 et L est un groupement aminé éventuellement quaternisé choisi parmi les groupements :
      -N(R²)-CH₂-CH₂-N(R²)₂;
      -N(R²)₂; -N⁺(R²)₃ Q⁻;
      -N⁺(R²)(H)₂Q⁻;
      -N⁺(R²)₂HQ⁻;
      -N(R²)-CH₂-CH₂-N⁺(R²)(H)₂Q⁻,
   dans lesquels R² désigne un atome d'hydrogène, un phényle, un benzyle, ou un radical hydrocarboné saturé monovalent, par exemple un radical alkyle en C₁-C₂₀, et Q⁻ représente un ion halogénure tel que par exemple fluorure, chlorure, bromure ou iodure.
      En particulier, les silicones aminées correspondant à la définition de la formule (I) sont choisies parmi les composés correspondant à la formule (II) suivante : 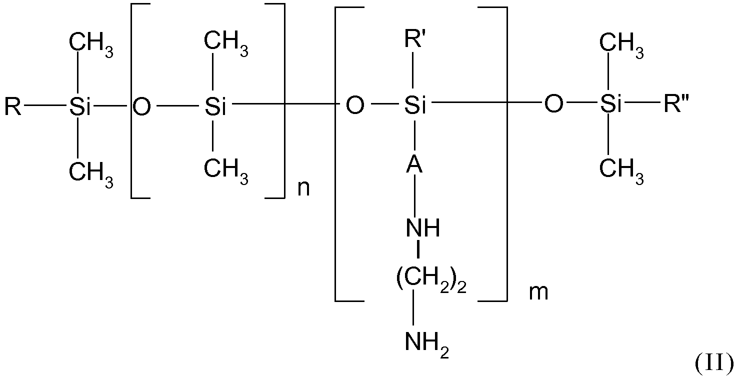 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.
      Selon une première possibilité, R, R', R", identiques ou différents, représentent un radical alkyle en C₁-C₄ ou hydroxyle, A représente un radical alkylène en C₃, et m et n sont tels que la masse moléculaire moyenne en poids du composé est comprise entre 5 000 et 500 000 environ. Des composés de ce type sont dénommés dans le dictionnaire CTFA, "amodiméthicone".
      Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.
      Dans cette catégorie de composés, on peut citer entre autres, le produit Belsil®ADM 652, commercialisée par Wacker.
      Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.
      Plus particulièrement, on peut citer le produit FluidWR® 1300, commercialisé par Wacker.
      Selon une quatrième possibilité, R, R" représentent un radical hydroxyle, R' représente un radical méthyle et A est un radical alkylène, en C₄-C₈, de préférence en C₄. Par ailleurs, m et n sont tels que la masse moléculaire moyenne en poids du composé est comprise entre 2000 et 10⁶. Plus particulièrement, n est compris entre 0 et 1999 et m est compris entre 1 et 2000, la somme de n et m étant comprise entre 1 et 2000.
      Un produit de ce type est notamment commercialisé sous la dénomination DC28299 par Dow Corning.
      Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).
      Un produit correspondant à la définition de la formule (I) est en particulier le polymère dénommé dans le dictionnaire CTFA (7^{ème} édition 1997) "triméthylsilylamodiméthicone", répondant à la formule (III) suivante: 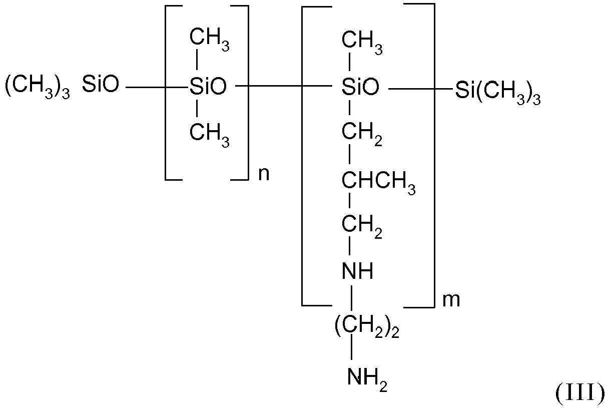 dans laquelle n et m ont les significations données ci-dessus conformément à la formule (I) ou (II).
      De tels composés sont décrits par exemple dans EP 0095238; un composé de formule (III) est par exemple vendu sous la dénomination Q2-8220 par la société OSI.
(b) les composés répondant à la formule (IV) suivante : 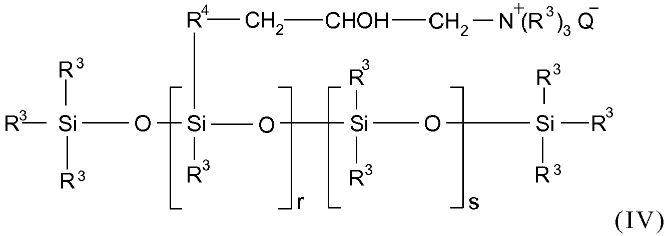 dans laquelle :
   R³ représente un radical hydrocarboné monovalent en C₁-C₁₈, et en particulier un radical alkyle en C₁-C₁₈ ou alcényle en C₂-C₁₈, par exemple méthyle ;
   R⁴ représente un radical hydrocarboné divalent, notamment un radical alkylène en C₁-C₁₈ ou un radical alkylèneoxy divalent en C₁-C₁₈, par exemple en C₁-C₈;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.
   De tels composés sont décrits plus particulièrement dans le brevet US 4185087.
   Un composé entrant dans cette classe est celui vendu par la Société Union Carbide sous la dénomination "Ucar Silicone ALE 56".
(c) les silicones ammonium quaternaire de formule (V) : 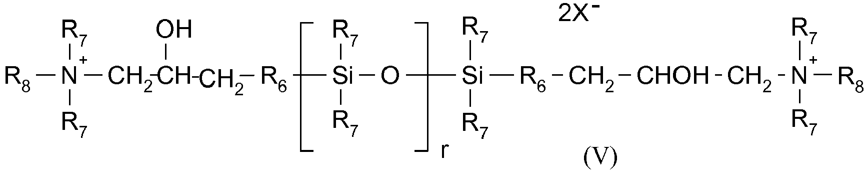 dans laquelle :
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
   X⁻ est un anion tel qu'un ion halogénure, notamment chlorure ou un sel d'acide organique (acétate ...);
   r représente une valeur statistique moyenne de 2 à 200 et en particulier de 5 à 100 ;
   Ces silicones sont par exemple décrites dans la demande EP-A-0530974.

   A titre de composé de formule (V) on peut citer le produit référencé dans le dictionnaire CTFA (Edition 1997) sous l'appellation Quaternium 80 tel que proposé par la Société EVONIK GOLDSCHMIDT sous les noms ABIL QUAT 3272 ou 3474.
d) les silicones aminées de formule suivante: 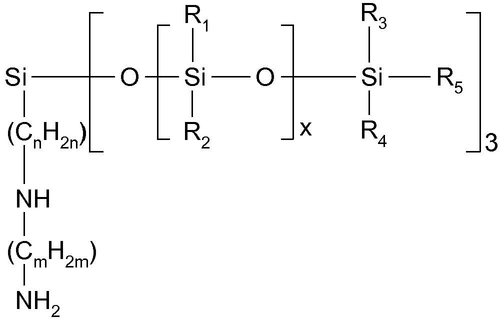 dans laquelle :
   - R₁, R₂, R₃ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5,
et dans laquelle x est choisi de manière telle que l'indice d'amine soit compris entre 0,01 et 1 meq/g.

La ou les silicones aminées particulièrement préférées sont les polysiloxanes à groupements aminés tels que les composés de formule (II) ou de formule (III), et encore plus particulièrement les silicones à groupements ammonium quaternaire de formule (V).

De préférence le ratio pondéral de la quantité d'alcane(s) linéaire(s) volatil(s) sur la quantité de silicone(s) aminée(s) varie de 0,5 à 100, plus préférentiellement de 0,5 à 50, et mieux encore de 1 à 20.

Lorsque ces composés sont mis en oeuvre, une forme de réalisation particulièrement intéressante est leur utilisation conjointe avec des agents de surface cationiques et/ou non ioniques.

On peut utiliser par exemple le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule : C₁₃H₂₇-(OC₂H₄)₁₂-OH, connu sous la dénomination CTFA "tridéceth-12".

Un autre produit commercial utilisable selon l'invention est le produit vendu sous la dénomination "Dow Corning Q2 7224" par la Société Dow Corning, comportant en association le triméthylsilylamodiméthicone de formule (C) décrite ci-dessus, un agent de surface non ionique de formule : C₈H₁₇-C₆H₄-(OCH₂CH₂)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.

La ou les silicones aminées sont présentes dans la composition selon l'invention en une quantité d'au moins 0,5% en poids, de préférence dans une quantité variant de 0,5 à 20% en poids, plus particulièrement en une quantité variant de 0,5 à 10% en poids, et mieux encore dans une quantité variant de 0,75 à 5% en poids, par rapport au poids total de la composition.

La composition selon l'invention contient une ou plusieurs huiles végétales.

On entend par "huile" tout composé lipophile, non ionique, insoluble dans l'eau et liquide à température ambiante (25 °C) et sous pression atmosphérique (760 mm de Hg, soit 101 325 Pa). Par insoluble dans l'eau, on entend au sens de la présente invention un composé dont la solubilité à pH spontané dans l'eau à 25 °C et à pression atmosphérique est inférieure à 1 %, et de préférence inférieure à 0,5 % en poids. Les huiles sont solubles dans des solvants organiques dans les mêmes conditions de température et de pression, comme par exemple le chloroforme, l'éthanol ou le benzène. De plus les huiles sont liquides à la température ordinaire (25°C) et à pression atmosphérique. Les huiles ont de préférence une température de fusion inférieure à 5°C et une viscosité inférieure à 500 cPs à 25 °C à un taux de cisaillement de 1s⁻¹.

En particulier, on entend par "huile végétale" une huile telle que définie ci-dessus, extraite d'une espèce appartenant au règne végétal.

L'huile végétale ou les huiles végétales utilisées selon l'invention sont différentes des alcanes linéaires volatils tels que définis ci-avant et sont choisies parmi les huiles végétales habituellement employées dans le domaine cosmétique.

A titre d'exemple d'huile végétale utilisable dans les compositions de l'invention, on peut citer :
- l'huile d'amande douce,
- l'huile d'argan,
- l'huile d'avocat,
- l'huile d'arachide,
- l'huile de camélia,
- l'huile de carthame,
- l'huile de calophyllum,
- l'huile de colza,
- l'huile de coprah,
- l'huile de coriandre,
- l'huile de courge,
- l'huile de germes de blé,
- l'huile de jojoba ou cire liquide de jojoba,
- l'huile de lin,
- l'huile de macadamia,
- l'huile de germes de maïs,
- l'huile de noisette,
- l'huile de noix,
- l'huile de vernonia,
- l'huile de noyau d'abricot,
- l'huile d'olive,
- l'huile d'onagre,
- l'huile de palme,
- l'huile de passiflore,
- l'huile de pépins de raisin,
- l'huile de rosier,
- l'huile de ricin,
- l'huile de seigle,
- l'huile de sésame,
- l'huile de son de riz,
- l'huile de soja, et
- l'huile de tournesol.

Les huiles végétales selon l'invention, généralement, n'ont pas subi de transformation chimique après extraction.

Parmi les huiles végétales citées ci-dessus, on utilise de préférence l'huile d'olive, l'huile d'argan, l'huile d'avocat, l'huile de colza, l'huile de jojoba ou cire liquide de jojoba, l'huile de soja, l'huile de tournesol, et de manière plus préférée l'huile d'avocat, l'huile de jojoba ou cire liquide de jojoba

De préférence, l'huile ou les huiles végétales sont présentes dans la composition dans une quantité variant de 0,3 à 30%, plus préférentiellement dans une quantité variant de 1 à 20%, et mieux encore dans une quantité variant de 3 à 15% en poids, par rapport au poids total de la composition.

La composition selon l'invention peut comprendre en outre un ou plusieurs tensioactifs choisis parmi des tensioactifs anioniques, amphotères ou zwittérioniques, non ioniques et cationiques.

Parmi les tensioactifs anioniques utilisables selon l'invention, on peut citer notamment les sels, en particulier les sels alcalins et notamment de sodium, les sels d'ammonium, les sels d'amines, les sels d'aminoalcools ou les sels de magnésium des composés suivants : les alkylsulfates, les alkyléthersulfates, les alkylamidoéthersulfates, les monoglycérides sulfates, les alkylglycérylsulfonates, les alkylsulfonates, les alkyl-phosphates, les alkylamidesulfonates, les alkylarylsulfonates, les α-oléfinesulfonates, les paraffines sulfonates, les alkylsulfosuccinates, les alkyléthersulfosuccinates, les alkylamidesulfosuccinates, les alkylsulfosuccinamates, les alkylsulfoacétates, les alkylétherphosphates, les acyliséthionates, les N-acyltaurates, les N-acylaminoacides tels que les N-acylsarcosinates et les N-acylglutamates. On peut également citer comme agents tensioactifs anioniques pouvant être utilisés dans les compositions selon l'invention, les sels d'acides gras tels que les sels des acides undécénylique, oléique, ricinoléique, palmitique et stéarique, les acides d'huile de coprah ou d'huile de coprah hydrogénée et les acyl-hydroxyacides tels que les acyl-lactylates. On peut également utiliser des tensioactifs faiblement anioniques tels que les acides d'alkyl D-galactoside uroniques et leurs sels ainsi que les acides alkyléthers alkylamidoéthercarboxyliques polyoxyalkylénés ou leurs sels, le radical alkyle ou acyle de ces différents composés comportant de préférence de 8 à 22 atomes de carbone et les dérivés anioniques d'alkyle (C₈-C₂₂) polyglycosides (sulfate, sulfosuccinate, phosphate, iséthionate, éthercarboxylate, carbonate).

Parmi les tensioactifs amphotères utilisables selon l'invention, on peut notamment citer les dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant tel que par exemple, un groupe carboxylate, sulfonate, sulfate, phosphate ou phosphonate. On peut encore citer parmi les tensioactifs amphotères ou zwittérioniques, les sulfobétaines, les alkylamidoalkylbétaïnes, les alkylamidoalkylsulfobétaines, les dérivés d'imidazolium tels que ceux d'amphocarboxyglycinate ou d'amphocarboxypropionate.

Parmi les tensioactifs non ioniques utilisables selon l'invention, on peut citer notamment les dérivés polyéthoxylés polypropoxylés ou polyglycérolés des alcools ou des alphadiols ou des alkylphénols ou des acides gras, ayant une chaîne grasse comportant de 8 à 28 atomes de carbone, le nombre de groupes d'oxyde d'éthylène ou de propylène pouvant aller de 2 à 50 et celui de glycérol notamment de 2 à 30. On peut également citer les copolymères d'oxyde d'éthylène et de propylène, les condensats d'oxyde d'éthylène et de propylène sur des alcools gras, les amides gras polyéthoxylés ayant de préférence de 2 à 30 moles d'oxyde d'éthylène, les amides gras polyglycérolés comportant en moyenne 1 à 5 groupes glycérol, les diglycolamides polyglycérolés, les esters d'acides gras du sorbitan éventuellement oxyéthylénés, les esters d'acides gras du saccharose, les esters d'acides gras polyoxyalkylénés, les alkylpolyglycosides éventuellement oxyalkylénés, les esters d'alkylglucosides, les dérivés de N-alkylglucamine et de N-acyl-méthylglucamine, les aldobionamides et les oxydes d'amine.

On entend par « tensioactif cationique », un tensioactif chargé positivement lorsqu'il est contenu dans la composition selon l'invention. Ce tensioactif peut porter une ou plusieurs charges permanentes positives ou contenir une ou plusieurs fonctions cationisables au sein de la composition selon l'invention.

Parmi les tensioactifs cationiques utilisables dans la composition selon l'invention, on peut notamment citer les amines grasses primaires, secondaires ou tertiaires, éventuellement polyoxyalkylénées, ou leurs sels, les sels d'ammonium quaternaire, et leurs mélanges.

A titre de sels d'ammonium quaternaires, on peut notamment citer, par exemple :
- ceux qui présentent la formule générale (VII) suivante : 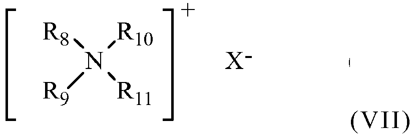 dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle, au moins un des radicaux R₈ à R₁₁ désignant un radical comportant de 8 à 30 atomes de carbone, de préférence de 12 à 24 atomes de carbone. Les radicaux aliphatiques peuvent comporter des hétéroatomes tels que notamment l'oxygène, l'azote, le soufre et les halogènes.
   Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline, comme par exemple ceux de formule (VIII) suivante : 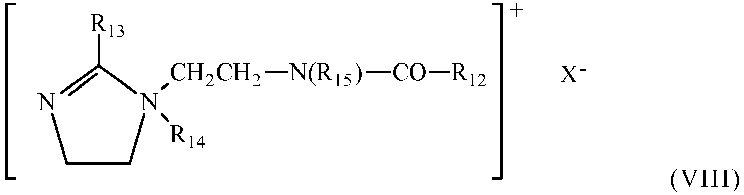 dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT^{®} W 75 par la société REWO ;
- les sels de diammonium quaternaire de formule (IX) : 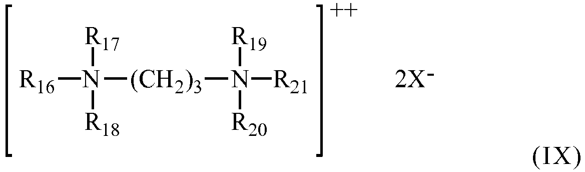 dans laquelle R₁₆ désigne un radical aliphatique comportant environ de 16 à 30 atomes de carbone, R₁₇, R₁₈, R₁₉, R₂₀ et R₂₁, identiques ou différents sont choisis parmi l'hydrogène ou un radical alkyle comportant de 1 à 4 atomes de carbone, et X⁻ est un anion choisi dans le groupe des halogénures, acétates, phosphates, nitrates et méthylsulfates. De tels sels de diammonium quaternaire comprennent notamment le dichlorure de propane suif diammonium ;
- les sels d'ammonium quaternaire contenant au moins une fonction ester, tels que ceux de formule (X) suivante : 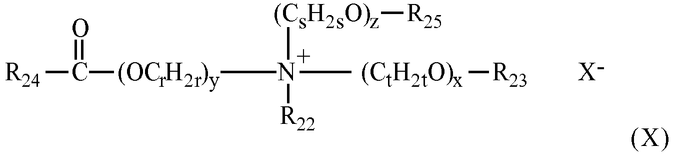 dans laquelle :
   R₂₂ est choisi parmi les radicaux alkyles en C₁-C₆ et les radicaux hydroxyalkyles ou dihydroxyalkyles en C₁-C₆ ;
   R₂₃ est choisi parmi :
      - le radical 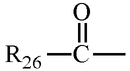
      - les radicaux R₂₇ qui sont des radicaux hydrocarbonés en C₁-C₂₂, linéaires ou ramifiés, saturés ou insaturés,
      - l'atome d'hydrogène,
   R₂₅ est choisi parmi :
      - le radical 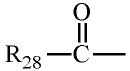
      - les radicaux R₂₉ qui sont des radicaux hydrocarbonés en C₁-C₆, linéaires ou ramifiés, saturés ou insaturés,
      - l'atome d'hydrogène,
   R₂₄, R₂₆ et R₂₈, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10 ;
   X⁻ est un anion simple ou complexe, organique ou inorganique ;
sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.
De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion X⁻ est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.
On utilise plus particulièrement dans la composition selon l'invention, les sels d'ammonium de formule (X) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 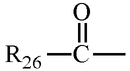
   - les radicaux méthyle, éthyle ou hydrocarbonés en C₁₄-C₂₂,
   - l'atome d'hydrogène ;
- R₂₅ est choisi parmi :
   - le radical 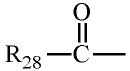
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires. On peut citer par exemple les composés de formule (X) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxy éthyl-méthylammonium, de monoacyloxyéthyl-hydroxyéthyl-diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthane sulfonate de méthyle, le paratoluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30% en poids de méthylsulfate d'acyloxyéthyl-dihydroxyéthyl-méthylammonium, 45 à 60% de méthyl sulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (VII), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le méthosulfate de distéaroyléthyl hydroxyéthyl méthylammonium, le méthosulfate de dipalmitoyléthylhydroxyéthylammonium ou le méthosulfate de dicétylaroyléthylhydroxyéthylammonium, ou encore, enfin, le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK.

Parmi tous les tensioactifs cationiques pouvant être présents dans la composition selon l'invention, on préfère choisir le ou les tensioactifs cationiques parmi les sels de (chlorure ou méthosulfate) de cétyltriméthylammonium (INCI cetrimonium-), de béhényltriméthylammonium (INCI behentrimonium-), de dipalmitoyléthylhydroxyéthylammonium, de distéaroyléthylhydroxyéthyl méthylammonium, de méthyl alkyl(C₉-C₁₉) alkyl(C₁₀-C₂₀)amidoéthylimidazolium, de stéaramidopropyldiméthylammonium, ou leurs mélanges.

Avantageusement, le ou les tensioactifs sont choisis parmi les tensioactifs non ioniques et les tensioactifs cationiques

Lorsque la composition comprend au moins un tensioactif, celui-ci est présent dans une concentration allant de préférence de 0,1 à 20% en poids, et plus préférentiellement de 0,2 à 10% en poids, du poids total de la composition.

La composition utilisée selon l'invention comprend un milieu cosmétiquement acceptable.

Le milieu cosmétiquement acceptable est constitué d'eau ou d'un mélange d'eau et d'au moins un solvant cosmétiquement acceptable de préférence choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

La composition selon l'invention peut comprendre en outre un ou plusieurs additifs classiques bien connus dans la technique, différents des composés définis précédemment. A titre d'exemples d'additifs utilisables selon l'invention, on peut citer des polymères associatifs ou non, ioniques ou non-ioniques et en particulier des polymères cationiques, des corps gras liquides ou solides différents des huiles végétales, des silicones non aminées, des silanes, des protéines, des vitamines, des agents réducteurs, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des agents nacrants, des propulseurs, et des épaississants minéraux ou organiques ; ces additifs étant différents des composés définis plus haut.

L'homme de métier veillera à choisir le ou les éventuels additifs et leur quantité de manière à ce qu'ils ne nuisent pas aux propriétés des compositions de la présente invention.

Le ou les additifs sont généralement présents dans la composition selon l'invention en une quantité allant de 0 à 20% en poids par rapport au poids total de la composition.

Les compositions selon l'invention peuvent se présenter sous la forme de composition de soin rincé ou non-rincé, ces dernières se présentant sous la forme d'une lotion plus ou moins épaissie, d'une crème, d'un gel ou d'une émulsion.

Un autre objet de l'invention est l'utilisation de la composition cosmétique telle que décrite ci-dessus pour le traitement cosmétique des matières kératiniques, de préférence des fibres kératiniques comme les cheveux, et notamment comme produit capillaire rincé.

L'invention est également relative à un procédé de traitement cosmétique des matières kératiniques, de préférence des fibres kératiniques comme les cheveux, qui comprend l'application d'une quantité efficace d'une composition cosmétique telle que décrite ci-dessus, sur lesdites matières, et éventuellement le rinçage de ladite composition après un éventuel temps de pause.

Lorsque l'on applique la composition selon l'invention sous forme d'une lotion ou d'une crème, on la laisse éventuellement pauser sur les cheveux pendant environ 0,5 à 5 minutes, puis on rince éventuellement à l'eau.

Les exemples suivants sont donnés à titre illustratif de la présente invention.

Dans les exemples suivants, toutes les quantités sont indiquées en pour cent en poids de produit en l'état par rapport au poids total de la composition, sauf indication contraire.

### EXEMPLE 1 - Comparaison

On a préparé les compositions de soin rincé A, B suivantes à partir des ingrédients indiqués dans le tableau ci-dessous.

| Compositions | A comparatif | B invention |
|---|---|---|
| Cyclopentadiméthylsiloxane (Xiameter PMX-0245 cyclosiloxane - Dow Corning) | 3 | - |
| Mélange de n-undécane et de n-tridécane selon l'exemple 2 de la demande WO 2008/155059 | - | 3 |
| Polydiméthylsiloxane à groupements aminoéthyliminopropyle en microémulsion non ionique à 17% en poids dans l'eau (Wacker Belsil ADM Log 1 - Wacker) | 5 | 5 |
| Cire liquide de jojoba | 6 | 6 |
| Huile d'avocat | 5 | 5 |
| Alcool éthylique à 96 degrés | 17 | 17 |
| Glycérol | 4 | 4 |
| Chlorure de béhényl triméthyl ammonium à 79% en poids de matière active (Genamin KDMP - Clariant) | 2 | 2 |
| Mono isostéarate de polyéthylène glycol à 8 moles d'oxyde d'éthylène | 2 | 2 |
| Parfum | qs | qs |
| Eau désionisée | Qs 100 | Qs 100 |

La composition A a été comparée à la composition B selon l'invention.

On a appliqué 6 grammes de composition A sur une demie-tête et 6 grammes de composition B sur l'autre demie-tête d'un modèle. Après malaxage des cheveux, on a rincé les cheveux à l'eau.

On a évalué la souplesse des cheveux lors du rinçage, le décollement des racines sur des cheveux humides et la vitesse de séchage des cheveux.

Cette évaluation a été réalisée sur un panel de 10 modèles par des experts entraînés régulièrement à cet exercice. Pour chaque critère évalué, des notes allant de 0 (pas bon) à 5 (très bon) ont été attribuées.

On a observé les résultats suivants :

**Comparaison des compositions A et B**

| | A comparatif | B invention |
|---|---|---|
| Souplesse de la fibre capillaire lors du rinçage sous l'eau | 3,5 | 4,0 |
| Décollement des racines sur cheveux humides | 1,4 | 2,0 |

La composition B selon l'invention conduit à un meilleur assouplissement de la fibre capillaire au moment du rinçage, et sur cheveux humides, à plus de volume (les racines sont plus décollées).

Il a également été observé un séchage plus rapide des cheveux traités avec la composition B par rapport à la composition comparative A.

### EXEMPLE 2 - Autre composition de soin rincé selon l'invention

| Composition | C invention |
|---|---|
| Mélange de n-dodécane et de n-tétradécane (VEGELIGHT 1214 - Biosynthis) | 3 |
| Polydiméthylsiloxane à groupements aminoéthyl-iminopropyle en microémulsion non ionique à 17% en poids dans l'eau (Wacker Belsil ADM Log 1 - Wacker) | 5 |
| Cire liquide de jojoba | 6 |
| Huile d'avocat | 5 |
| Alcool éthylique à 96 degrés | 17 |
| Glycérol | 4 |
| Chlorure de béhényl triméthyl ammonium à 79% en poids de matière active (Genamin KDMP - Clariant) | 2 |
| Mono iosostéarate de polyéthylène glycol à 8 moles d'oxyde d'éthylène | 2 |
| Parfum | qs |
| Eau désionisée | Qs 100 |

Les cheveux traités avec la composition C présentent les mêmes propriétés que ceux traités avec la composition B.

## Revendications

1. Composition cosmétique comprenant dans un milieu cosmétiquement acceptable :
- de 3 à 30 % en poids d'un ou plusieurs alcanes linéaires volatils comportant de 7 à 15 atomes de carbone par rapport au poids total de ladite composition,
- au moins 0,5% en poids d'une ou plusieurs silicones aminées par rapport au poids total de ladite composition,
- de 3 à 20 % en poids d'une ou plusieurs huiles végétales différentes des alcanes linéaires volatils par rapport au poids total de ladite composition.

2. Composition cosmétique selon la revendication 1, **caractérisée en ce que** le ou les alcanes linéaires volatils sont choisis parmi le n-heptane (C7), le n-octane (C8), le n-nonane (C9), le n-décane (C10), le n-undécane (C11), le n-dodécane (C12), le n-tridécane (C13), le n-tétradecane (C14), et leurs mélanges.

3. Composition cosmétique selon l'une quelconque des revendications précédentes, **caractérisée en ce que** le ou les alcanes linéaires volatils sont d'origine végétale.

4. Composition cosmétique selon l'une quelconque des revendications précédentes, **caractérisée en ce que** la ou les silicones aminées sont choisies parmi :
(a) les composés répondant à la formule (I) suivante :
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (I)
dans laquelle :
T est un atome d'hydrogène, ou un radical phényle, hydroxyle (-OH), ou alkyle en C₁-C₈, a désigne le nombre 0 ou un nombre entier de 1 à 3, b désigne 0 ou 1,
m et n sont des nombres tels que la somme (n + m) peut varier de 1 à 2 000, n pouvant désigner un nombre de 0 à 1 999 et m pouvant désigner un nombre de 1 à 2 000;
R¹ est un radical monovalent de formule -C_{q}H_{2q}L dans laquelle q est un nombre de 2 à 8 et L est un groupement aminé éventuellement quaternisé choisi parmi les groupements :
- N(R²)-CH₂-CH₂-N(R²)₂ ;
- N(R²)₂ ; -N⁺(R²)₃Q⁻ ;
- N⁺(R²)(H)₂Q⁻ ;
- N⁺(R²)₂HQ⁻ ;
- N(R²)-CH₂-CH₂-N⁺(R²)(H)₂Q⁻,
dans lesquels R² désigne un atome d'hydrogène, un phényle, un benzyle, ou un radical hydrocarboné saturé monovalent, et Q⁻ représente un ion halogénure.
(b) les composés répondant à la formule (IV) suivante : 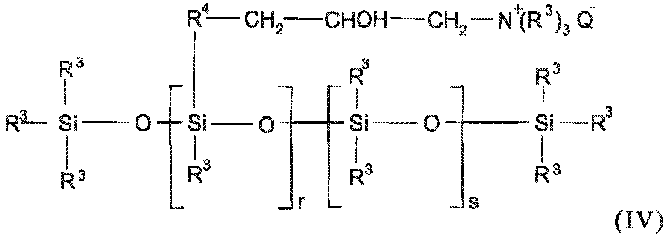 dans laquelle :
R³ représente un radical hydrocarboné monovalent en C₁-C₁₈;
R⁴ représente un radical hydrocarboné divalent;
Q⁻ est un ion halogénure;
r représente une valeur statistique moyenne de 2 à 20;
s représente une valeur statistique moyenne de 20 à 200.
(c) les silicones ammonium quaternaire de formule (V) : 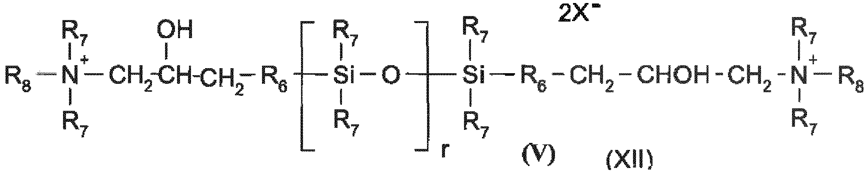 dans laquelle :
R₇, identiques ou différents, représentent un radical hydrocarboné monovalent ayant de 1 à 18 atomes de carbone;
R₆ représente un radical hydrocarboné divalent ou un radical alkylèneoxy divalent en C₁-C₁₈ relié au Si par une liaison SiC;
R₈, identiques ou différents, représentent un atome d'hydrogène, un radical hydrocarboné monovalent ayant de 1 à 18 atomes de carbone, un radical -R₆-NHCOR₇;
X⁻ est un anion;
r représente une valeur statistique moyenne de 2 à 200.
d) les silicones aminées de formule suivante: 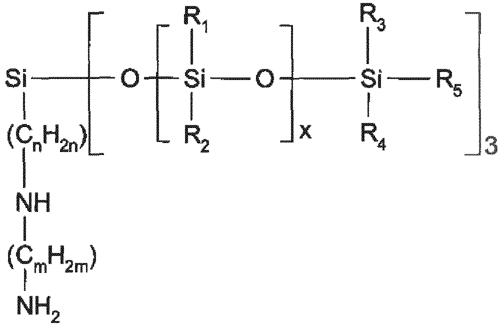 dans laquelle :
- R₁, R₂, R₃ et R₄, identiques ou différents, désignent un radical alkyle en C₁-C₄ ou un groupement phényle,
- R₅ désigne un radical alkyle en C₁-C₄ ou un groupement hydroxyle,
- n est un entier variant de 1 à 5,
- m est un entier variant de 1 à 5,
et dans laquelle x est choisi de manière telle que l'indice d'amine soit compris entre 0,01 et 1 meq/g.

5. Composition cosmétique selon la revendication précédente, **caractérisée en ce que** la ou les silicones aminées de formule (I) sont choisies parmi les composés correspondant à la formule (II) suivante : 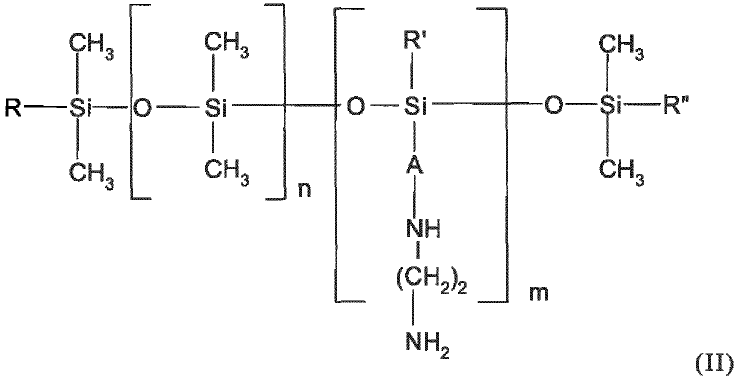 dans laquelle R, R', R", identiques ou différents, désignent un radical alkyle en C₁-C₄; un radical alcoxy en C₁-C₄; ou OH; A représente un radical alkylène, linéaire ou ramifié, en C₃-C₈; et m et n sont des nombres entiers dépendant du poids moléculaire et dont la somme est comprise entre 1 et 2000.

6. Composition cosmétique selon la revendication précédente, **caractérisée en ce que** R, R', R", A et m et n sont définis selon l'une des possibilités suivantes:
(i) R, R', R", identiques ou différents, représentent un radical alkyle en C₁-C₄ ou hydroxyle, A représente un radical alkylène en C₃, et m et n sont tels que la masse moléculaire moyenne en poids du composé est comprise entre 5 000 et 500 000 environ.
(ii) R, R', R", identiques ou différents, représentent un radical alcoxy en C₁-C₄ ou hydroxyle, l'un au moins des radicaux R ou R" étant un radical alcoxy, A représente un radical alkylène en C₃, et m et n sont tels que la masse moléculaire moyenne en poids du composé est comprise entre 2000 et 10⁶.
(iii) R, R", différents, représentent un radical alcoxy en C₁-C₄ ou hydroxyle, l'un au moins des radicaux R, R" étant un radical alcoxy, R' représente un radical méthyle, A représente un radical alkylène en C₃, et m et n sont tels que la masse moléculaire moyenne en poids du composé est comprise entre 2000 et 200000.
(iv) R, R" représentent un radical hydroxyle, R' représente un radical méthyle, A est un radical alkylène en C₄-C₈, et m et n sont tels que la masse moléculaire moyenne en poids du composé est comprise entre 2000 et 10⁶

7. Composition cosmétique selon l'une quelconques des revendications 4 à 6, **caractérisée en ce que** la ou les silicones aminées de formule (I) sont choisies parmi les composés correspondant à la formule (III) suivante: 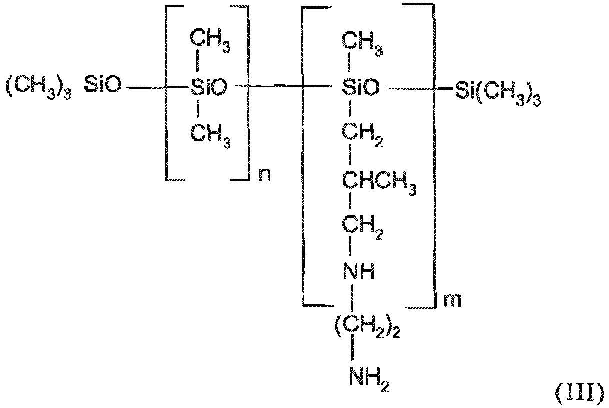 dans laquelle n et m sont tels que définis dans l'une quelconque des revendications 4 à 6.

8. Composition cosmétique selon l'une quelconque des revendications 4 à 7, **caractérisée en ce que** la ou les silicones aminées sont choisies parmi les silicones de formule (II), (III) et (V).

9. Composition cosmétique selon l'une quelconque des revendications précédentes, **caractérisée en ce que** la ou les silicones aminées sont présentes en une teneur allant de 0,5 à 20%, plus particulièrement allant de 0,5 à 10%, et mieux encore de 0,75 à 5% en poids, par rapport au poids total de la composition.

10. Composition cosmétique selon l'une quelconque des revendications précédentes, **caractérisée en ce que** l'huile ou les huiles végétales sont choisies parmi :
- l'huile d'amande douce,
- l'huile d'argan,
- l'huile d'avocat,
- l'huile d'arachide,
- l'huile de camélia,
- l'huile de carthame,
- l'huile de calophyllum,
- l'huile de colza,
- l'huile de coprah,
- l'huile de coriandre,
- l'huile de courge,
- l'huile de germes de blé,
- l'huile de jojoba ou cire liquide de jojoba,
- l'huile de lin,
- l'huile de macadamia,
- l'huile de germes de maïs,
- l'huile de noisette,
- l'huile de noix,
- l'huile de vernonia,
- l'huile de noyau d'abricot,
- l'huile d'olive,
- l'huile d'onagre,
- l'huile de palme,
- l'huile de passiflore,
- l'huile de pépins de raisin,
- l'huile de rosier,
- l'huile de ricin,
- l'huile de seigle,
- l'huile de sésame,
- l'huile de son de riz,
- l'huile de soja, et
- l'huile de tournesol
et de préférence parmi l'huile d'olive, l'huile d'argan, l'huile d'avocat, l'huile de colza, l'huile de jojoba ou cire liquide de jojoba, l'huile de soja et l'huile de tournesol.

11. Composition cosmétique selon l'une quelconque des revendications précédentes, **caractérisée en ce que** l'huile ou les huiles végétales sont présentes en une teneur allant de 3 à 15% en poids, par rapport au poids total de la composition.

12. Utilisation de la composition cosmétique selon l'une quelconque des revendications précédentes, pour le traitement des matières kératiniques, de préférence des cheveux.

13. Procédé de traitement cosmétique des matières kératiniques, de préférence des fibres kératiniques, et mieux encore des cheveux, comprenant l'application de la composition cosmétique selon l'une quelconque des revendications 1 à 11.

## Patentansprüche

1. Kosmetische Zusammensetzung, umfassend in einem kosmetisch unbedenklichen Medium:
- 3 bis 30 Gew.-% eines oder mehrerer flüchtiger linearer Alkane mit 7 bis 15 Kohlenstoffatomen, bezogen auf das Gesamtgewicht der Zusammensetzung,
- mindestens 0,5 Gew.-% eines oder mehrerer Aminosilikone, bezogen auf das Gesamtgewicht der Zusammensetzung,
- 3 bis 20 Gew.-% eines oder mehrerer pflanzlicher Öle, die von flüchtigen linearen Alkanen verschieden sind, bezogen auf das Gesamtgewicht der Zusammensetzung.

2. Kosmetische Zusammensetzung nach Anspruch 1, **dadurch gekennzeichnet, dass** das bzw. die flüchtigen linearen Alkane aus n-Heptan (C7), n-Octan (C8), n-Nonan (C9), n-Decan (C10), n-Undecan (C11), n-Dodecan (C12), n-Tridecan (C13), n-Tetradecan (C14) und Mischungen davon ausgewählt sind.

3. Kosmetische Zusammensetzung nach einem der vorhergehenden Ansprüche, **dadurch gekennzeichnet, dass** das bzw. die flüchtigen linearen Alkane pflanzlichen Ursprungs sind.

4. Kosmetische Zusammensetzung nach einem der vorhergehenden Ansprüche, **dadurch gekennzeichnet, dass** das bzw. die Aminosilikone ausgewählt sind aus:
(a) Verbindungen, die der folgenden Formel (I) entsprechen:
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (I)
worin:
T für ein Wasserstoffatom oder einen Phenyl-, Hydroxyl- (-OH-) oder C₁-C₈-Alkylrest steht, a für die Zahl 0 oder eine ganze Zahl von 1 bis 3 steht, b für 0 oder 1 steht,
m und n für solche Zahlen stehen, dass die Summe (n + m) von 1 bis 2000 variieren kann, wobei n für eine Zahl von 0 bis 1999 stehen kann und m für eine Zahl von 1 bis 2000 stehen kann;
R¹ für einen einwertigen Rest der Formel -C_{q}H_{2q}L steht, wobei q für eine Zahl von 2 bis 8 steht und
L für eine gegebenenfalls quaternisierte Aminogruppe steht, die aus den folgenden Gruppen ausgewählt ist:
- N(R²)-CH₂-CH₂-N(R²)₂;
- N(R²)₂; -N⁺(R²)₃Q⁻;
- N⁺(R²)(H)₂Q⁻;
- N⁺(R²)₂HQ⁻;
- N(R²)-CH₂-CH₂-N⁺(R²)(H)₂Q⁻,
wobei R² für ein Wasserstoffatom, ein Phenyl, ein Benzyl oder einen einwertigen gesättigten Kohlenwasserstoffrest steht und Q- für ein Halogenidion steht;
(b) Verbindungen, die der folgenden Formel (IV) entsprechen: 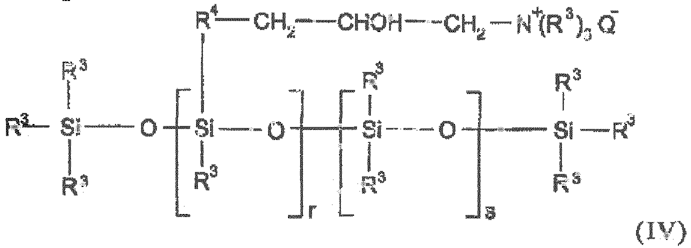 worin:
R³ für einen einwertigen C₁-C₁₈-Kohlenwasserstoffrest steht;
R⁴ für einen zweiwertigen Kohlenwasserstoffrest steht;
Q⁻ für ein Halogenidion steht;
r für einen durchschnittlichen statistischen Wert von 2 bis 20 steht;
s für einen durchschnittlichen statistischen Wert von 20 bis 200 steht;
(c) quaternären Ammoniumsilikonen der Formel (V): 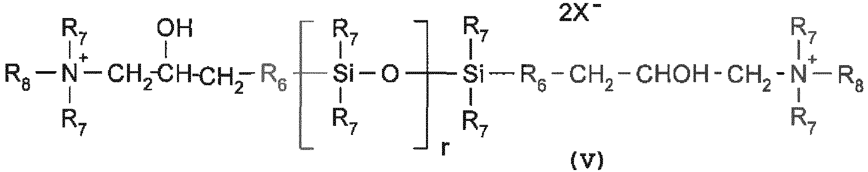 worin:
die Variablen R₇ gleich oder verschieden sind und für einen einwertigen Kohlenwasserstoffrest mit 1 bis 18 Kohlenstoffatomen stehen;
R₆ für einen zweiwertigen Kohlenwasserstoffrest oder einen zweiwertigen C₁-C₁₈-Alkylenoxyrest steht, der über eine SiC-Bindung an das Si gebunden ist;
die Variablen R₈ gleich oder verschieden sind und für ein Wasserstoffatom, einen einwertigen Kohlenwasserstoffrest mit 1 bis 18 Kohlenstoffatomen oder einen -R₆-NHCOR₇-Rest stehen;
X- für ein Anion steht,
r für einen durchschnittlichen statistischen Wert von 2 bis 200 steht;
d) Aminosilikonen der folgenden Formel: 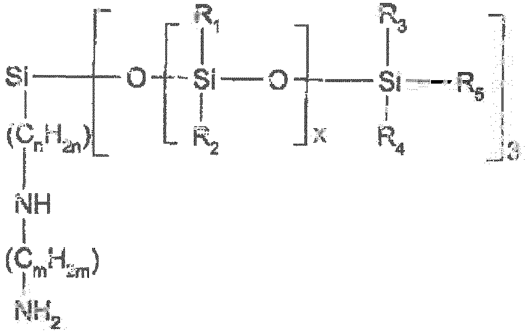 worin:
R₁, R₂, R₃ und R₄ gleich oder verschieden sind und für einen C₁-C₄-Alkylrest oder eine Phenylgruppe stehen,
R₅ für einen C₁-C₄-Alkylrest oder eine Hydroxylgruppe steht,
n für eine ganze Zahl im Bereich von 1 bis 5 steht,
m für eine ganze Zahl im Bereich von 1 bis 5 steht und x so gewählt ist, dass die Aminzahl im Bereich von 0,01 bis 1 meq/g liegt.

5. Kosmetische Zusammensetzung nach dem vorhergehenden Anspruch, **dadurch gekennzeichnet, dass** das bzw. die Aminosilikone der Formel (I) aus den Verbindungen, die der folgenden Formel (II) entsprechen, ausgewählt sind: 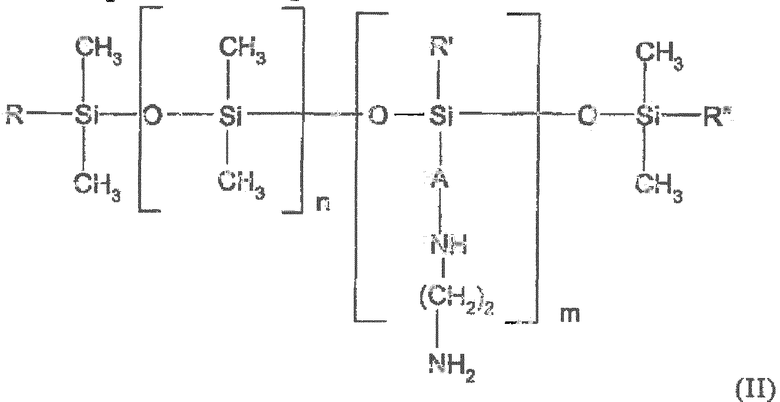 worin:
R, R' und R'' gleich oder verschieden sind und für einen C₁-C₄-Alkylrest; einen C₁-C₄-Alkoxyrest oder OH stehen; A für einen linearen oder verzweigten C₃-C₈-Alkylenrest steht und m und n ganze Zahlen sind, die vom Molekulargewicht abhängen und deren Summe zwischen 1 und 2000 liegt.

6. Kosmetische Zusammensetzung nach dem vorhergehenden Anspruch, **dadurch gekennzeichnet, dass** R, R', R", A und m und n gemäß einer der folgenden Möglichkeiten definiert sind:
(i) R, R' und R" sind gleich oder verschieden und stehen für einen C₁-C₄-Alkyl- oder Hydroxylrest, A steht für einen C₃-Alkylenrest, und m und n sind so beschaffen, dass das gewichtsmittlere Molekulargewicht der Verbindung zwischen etwa 5000 und 500.000 liegt;
(ii) R, R' und R" sind gleich oder verschieden und stehen für einen C₁-C₄-Alkoxy- oder Hydroxylrest, wobei mindestens einer der Reste R oder R" für einen Alkoxyrest steht, A steht für einen C₃-Alkylenrest, und m und n sind so beschaffen, dass das gewichtsmittlere Molekulargewicht der Verbindung zwischen 2000 und 10⁶ liegt;
(iii) R und R" sind verschieden und stehen für einen C₁-C₄-Alkoxy- oder Hydroxylrest, wobei mindestens einer der Reste R oder R" für einen Alkoxyrest steht, R' steht für einen Methylrest, A steht für einen C₃-Alkylenrest, und m und n sind so beschaffen, dass das gewichtsmittlere Molekulargewicht der Verbindung zwischen 2000 und 200.000 liegt;
(iv) R und R" stehen für einen Hydroxylrest, R' steht für einen Methylrest, A steht für einen C₄-C₈-Alkylenrest, und m und n sind so beschaffen, dass das gewichtsmittlere Molekulargewicht der Verbindung zwischen 2000 und 10⁶ liegt.

7. Kosmetische Zusammensetzung nach einem der Ansprüche 4 bis 6, **dadurch gekennzeichnet, dass** das bzw. die Aminosilikone der Formel (I) aus den Verbindungen, die der folgenden Formel (III) entsprechen, ausgewählt sind: 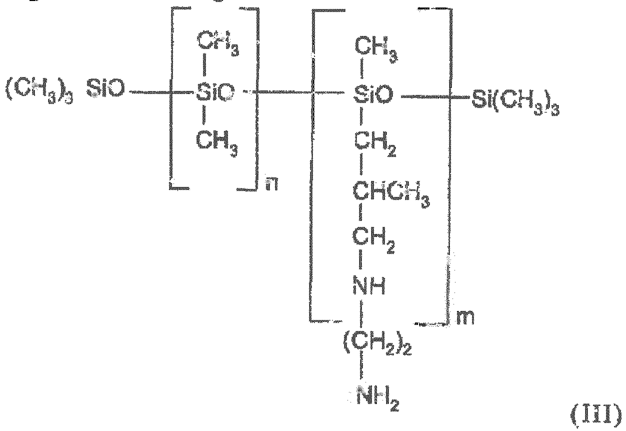 worin n und m wie in einem der Ansprüche 4 bis 6 definiert sind.

8. Kosmetische Zusammensetzung nach einem der Ansprüche 4 bis 7, **dadurch gekennzeichnet, dass** das bzw. die Aminosilikone aus den Silikonen der Formel (II), (III) und (V) ausgewählt sind.

9. Kosmetische Zusammensetzung nach einem der vorhergehenden Ansprüche, **dadurch gekennzeichnet, dass** das bzw. die Aminosilikone in einem Gehalt im Bereich von 0,5 bis 20 Gew.-%, spezieller im Bereich von 0,5 bis 10 Gew. -% und noch besser von 0,75 bis 5 Gew.-%, bezogen auf das Gesamtgewicht der Zusammensetzung, vorliegen.

10. Kosmetische Zusammensetzung nach einem der vorhergehenden Ansprüche, **dadurch gekennzeichnet, dass** das pflanzliche Öl bzw. die pflanzlichen Öle ausgewählt sind aus:
- Süßmandelöl,
- Arganöl,
- Avocadoöl,
- Erdnussöl,
- Kamelienöl,
- Saffloröl,
- Calophyllumöl,
- Colzaöl,
- Copraöl,
- Korianderöl,
- Kürbisöl,
- Weizenkeimöl,
- Jojobaöl oder Jojobaflüssigwachs,
- Leinsamenöl,
- Macadamiaöl,
- Maiskeimöl,
- Haselnussöl,
- Walnussöl,
- Vernoniaöl,
- Aprikosenkernöl,
- Olivenöl,
- Nachtkerzenöl,
- Palmöl,
- Passionsblumenöl,
- Traubenkernöl,
- Rosenöl,
- Ricinusöl,
- Roggenöl,
- Sesamöl,
- Reiskleieöl,
- Sojaöl und
- Sonnenblumenöl
und vorzugsweise aus Olivenöl, Arganöl, Avocadoöl, Colzaöl, Jojobaöl oder Jojobaflüssigwachs, Sojaöl und Sonnenblumenöl.

11. Kosmetische Zusammensetzung nach einem der vorhergehenden Ansprüche, **dadurch gekennzeichnet, dass** das pflanzliche Öl bzw. die pflanzlichen Öle in einem Gehalt im Bereich von 3 bis 15 Gew.-%, bezogen auf das Gesamtgewicht der Zusammensetzung, vorliegen.

12. Verwendung der kosmetischen Zusammensetzung nach einem der vorhergehenden Ansprüche zur Behandlung von Keratinmaterialien, vorzugsweise der Haare.

13. Verfahren zur kosmetischen Behandlung von Keratinmaterialien, vorzugsweise von Keratinfasern und noch besser der Haare, umfassend das Aufbringen der kosmetischen Zusammensetzung nach einem der Ansprüche 1 bis 11.

## Claims

1. Cosmetic composition comprising, in a cosmetically acceptable medium:
- from 3 to 30 wt.% of one or more volatile linear alkanes having from 7 to 15 carbon atoms relative to the total weight of said composition,
- at least 0.5 wit.% of one or more aminated silicones relative to the total weight of said composition,
- from 3 to 20 wt.% of one or more vegetable oils different from the volatile linear alkanes relative to the total weight of said composition.

2. Cosmetic composition according to Claim 1, **characterized in that** the volatile linear alkane or alkanes are selected from n-heptane (C7), n-octane (C8), n-nonane (C9), n-decane (C10), n-undecane (C11), n-dodecane (C12), n-tridecane (C13), n-tetradecane (C14), and mixtures thereof.

3. Cosmetic composition according to either of the preceding claims, **characterized in that** the volatile linear alkane or alkanes are of vegetable origin.

4. Cosmetic composition according to any one of the preceding claims, **characterized in that** the aminated silicone or aminated silicones are selected from:
(a) the compounds corresponding to the following formula (I) :
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (I)
in which:
T is a hydrogen atom, or a phenyl, hydroxyl (-OH), or C₁-C₈ alkyl radical, a denotes the number 0 or an integer from 1 to 3, b denotes 0 or 1,
m and n are numbers such that the sum (n + m) can vary from 1 to 2000, with n denoting a number from 0 to 1999 and m denoting a number from 1 to 2000;
R¹ is a monovalent radical of formula -C_{q}H_{2q}L in which q is a number from 2 to 8 and L is an amino group optionally quaternized selected from the groups:
-N(R²)-CH₂-CH₂-N(R²)₂;
-N(R²)₂; -N⁺(R²)₃Q⁻;
-N+(R²)(H)₂Q⁻;
-N⁺(R²)₂HQ⁻;
-N(R²)-CH₂-CH₂-N⁺(R²)(H)₂Q⁻.
in which R² denotes a hydrogen atom, a phenyl, a benzyl, or a saturated monovalent hydrocarbon radical, and Q⁻ represents a halide ion.
(b) the compounds corresponding to the following formula (IV): 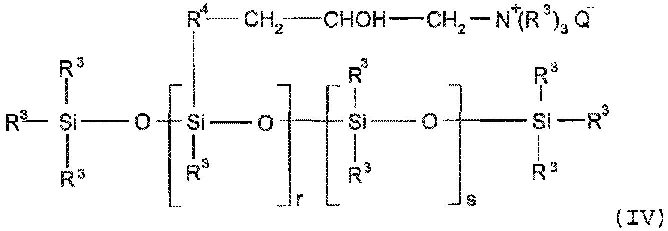 in which:
R³ represents a monovalent C₁-C₁₈ hydrocarbon radical;
R⁴ represents a divalent hydrocarbon radical;
Q⁻ is a halide ion;
r represents an average random value from 2 to 20;
s represents an average random value from 20 to 200.
(c) the quaternary ammonium silicones of formula (V): 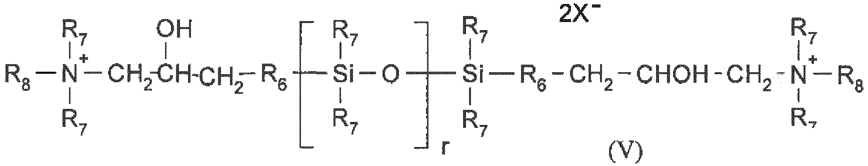 in which:
R₇, which may be identical or different, represent a monovalent hydrocarbon radical having from 1 to 18 carbon atoms;
R₆ represents a divalent hydrocarbon radical or a divalent C₁-C₁₈ alkyleneoxy radical joined to the Si by an SiC bond;
R₈, which may be identical or different, represent a hydrogen atom, a monovalent hydrocarbon radical having from 1 to 18 carbon atoms, a radical -R₆-NHCOR₇;
X⁻ is an anion;
r represents an average random value from 2 to 200.
d) the aminated silicones of the following formula: 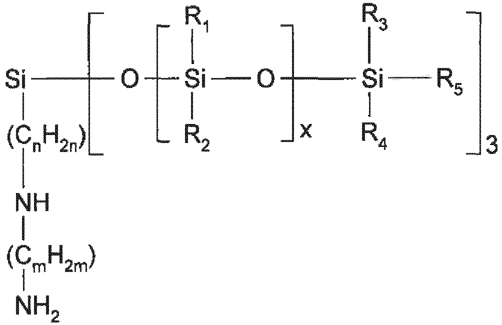 in which:
- R₁, R₂, R₃ and R₄, which may be identical or different, denote a C₁-C₄ alkyl radical or a phenyl group,
- R₅ denotes a C₁-C₄ alkyl radical or a hydroxyl group,
- n is an integer in the range from 1 to 5,
- m is an integer in the range from 1 to 5,
and in which x is selected in such a way that the amine index is between 0.01 and 1 meq/g.

5. Cosmetic composition according to the preceding claim, **characterized in that** the aminated silicone or aminated silicones of formula (I) are selected from the compounds corresponding to the following formula (II): 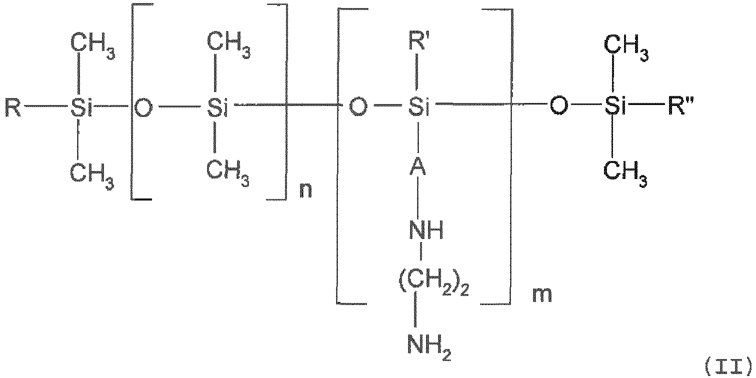 in which R, R', R", which may be identical or different, denote a C₁-C₄ alkyl radical; a C₁-C₄ alkoxy radical; or OH; A represents a linear or branched C₃-C₈ alkylene radical; and m and n are integers that depend on the molecular weight and whose sum is between 1 and 2000.

6. Cosmetic composition according to the preceding claim, **characterized in that** R, R', R", A and m and n are defined according to one of the following possibilities:
(i) R, R', R", which may be identical or different, represent a C₁-C₄ alkyl radical or a hydroxyl, A represents a C₃ alkylene radical, and m and n are such that the weight-average molecular weight of the compound is between about 5000 and 500 000.
(ii) R, R', R", which may be identical or different, represent a C₁-C₄ alkoxy radical or hydroxyl radical, at least one of the radicals R or R" being an alkoxy radical, A represents a C₃ alkylene radical, and m and n are such that the weight-average molecular weight of the compound is between 2000 and 10⁶.
(iii) R, R", which are different, represent a C₁-C₄ alkoxy radical or hydroxyl radical, at least one of the radicals R, R" being an alkoxy radical, R' represents a methyl radical, A represents a C₃ alkylene radical, and m and n are such that the weight-average molecular weight of the compound is between 2000 and 200000.
(iv) R, R" represent a hydroxyl radical, R' represents a methyl radical, A is a C₄-C₈ alkylene radical, and m and n are such that the weight-average molecular weight of the compound is between 2000 and 10⁶.

7. Cosmetic composition according to any one of Claims 4 to 6, **characterized in that** the aminated silicone or aminated silicones of formula (I) are selected from the compounds corresponding to the following formula (III): 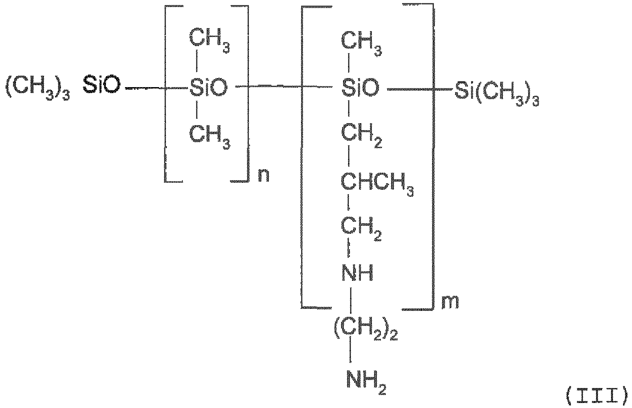 in which n and m are as defined in any one of Claims 4 to 6.

8. Cosmetic composition according to any one of Claims 4 to 7, **characterized in that** the aminated silicone or aminated silicones are selected from the silicones of formula (II), (III) and (V).

9. Cosmetic composition according to any one of the preceding claims, **characterized in that** the aminated silicone or aminated silicones are present at a content in the range from 0.5 to 20%, more particularly in the range from 0.5 to 10%, and better still from 0.75 to 5 wt.%, relative to the total weight of the composition.

10. Cosmetic composition according to any one of the preceding claims, **characterized in that** the vegetable oil or oils are selected from:
- sweet almond oil,
- argan oil,
- avocado oil,
- peanut oil,
- camellia oil,
- safflower oil,
- calophyllum oil,
- colza oil,
- copra oil,
- coriander oil,
- cucurbit oil,
- wheatgerm oil,
- jojoba oil or jojoba liquid wax,
- linseed oil,
- macadamia oil,
- maize germ oil,
- hazelnut oil,
- walnut oil,
- vernonia oil,
- apricot kernel oil,
- olive oil,
- evening primrose oil,
- palm oil,
- passionflower oil,
- grapeseed oil,
- rose oil,
- castor oil,
- rye oil,
- sesame oil,
- rice bran oil,
- soya oil, and
- sunflower oil
and preferably from olive oil, argan oil, avocado oil, colza oil, jojoba oil or jojoba liquid wax, soya oil and sunflower oil.

11. Cosmetic composition according to any one of the preceding claims, **characterized in that** the vegetable oil or oils are present at a content in the range from 3 to 15 wt.%, relative to the total weight of the composition.

12. Use of the cosmetic composition according to any one of the preceding claims, for the treatment of keratinous materials, preferably of the hair.

13. Method of cosmetic treatment of keratinous materials, preferably of keratin fibres, and more preferably of the hair, comprising the application of the cosmetic composition according to any one of Claims 1 to 11.
